# EUROPEAN PATENT APPLICATION

(11) **EP 1 941 876 A1**
(43) Date of publication of application: **09.07.2008**
(21) Application number: 06380338.1
(22) Date of filing: 28.12.2006
(51) Int. Cl.: A61K 31/34, A61P 27/00, A61P 27/06, A61P 29/00, A61P 43/00

(54) **Isosorbide mononitrate derivatives for the treatment of Inflammation and ocular hypertension**

(71) Applicant: LACER, S.A., 08025 Barcelona (ES)
(72) Inventor: Repollés Moliner, José, 08025 Barcelona (ES); Pubill Coy, Francisco, 08025 Barcelona (ES); Mourelle Mancini, Marisabel, 08025 Barcelona (ES)
(74) Representative: ABG Patentes, S.L.

(57) **Abstract**

The present invention relates to the use of a compound of formula (I) or a tautomer, a pharmaceutically acceptable salt, a prodrug or a solvate thereof: 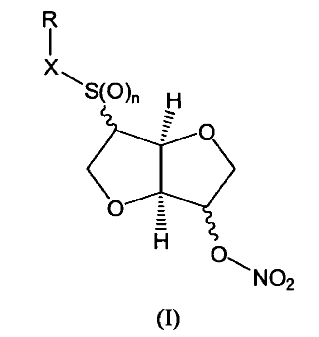 as active ingredient(s) in the manufacture of a pharmaceutical composition for the prevention and/or treatment of an ophthalmological disease mediated by ocular hypertension and/or an intestinal disorder.

## Description

### FIELD OF THE INVENTION

The present invention relates to the use of disulfide, sulfide, sulfoxide and sulfone derivatives of dianhydrohexite mononitrate of formula (I), tautomers, pharmaceutically acceptable salts, prodrugs and solvates thereof in the prevention and/or treatment of an ophthalmological disease mediated by ocular hypertension and intestinal disorders.

### BACKGROUND

Glaucoma is a degenerative disease of the eye wherein the intraocular pressure is too high to permit normal eye function. Pressure is elevated because drainage of aqueous fluid from within the eye is impaired. As a result, damage may occur to the optic nerve head and result in irreversible loss of visual function. If untreated, glaucoma may eventually lead to blindness. The several morphologically or functionally distinct types of glaucoma are typically characterized by elevated intraocular pressure (IOP), i.e., the condition of elevated intraocular pressure without optic nerve head damage or characteristic glaucomatous visual field defects, which is considered to be causally related to the pathological course of the disease. Some patients with glaucomatous field loss have relatively low IOP. These normal tension or low tension glaucoma patients can also benefit from agents that lower or control IOP. If glaucoma or ocular hypertension is detected early and treated promptly with medications that effectively reduce elevated intraocular pressure, the loss of visual function or its progressive deterioration can generally be ameliorated.

Current treatments for glaucoma focus on reducing pressure in the eye by reducing the amount of aqueous fluid being produced or by enhancing the flow of fluid out of the eye by mechanical or other means. However, currently available drugs do not enhance or restore functioning of the natural drainage pathway.

Glaucoma patients may also suffer reduced blood flow to the optic nerve and neuronal tissue, diminished resistance of the nerve tissue to damage, and compliance of connective tissue surrounding and supporting the optic nerve. Current treatments of do not address any such factors. Only, one agent, Memantine, has been proved to be an agent that may increase the relative resistance of the nerve tissue to damage (i.e., neuroprotective).

The use of statins has been associated in some studies with a diminished risk of developing age-related macular degeneration. To the extent that excess total cholesterol or LDL cholesterol is implicated in this condition, use of statins would reduce the risk of developing, or at least delay the onset of, this condition. Many statins also inhibit the activity of rho-kinase, such inhibition has been shown to enhance aqueous outflow [Rao et al., Invest. Ophthalmol. & Vis. Sci. 42:1029-1037 (2001)]. There may be as yet undiscovered or indirect effects of these compounds that would help explain their protective associations.

Recently, potassium channel blockers were found to reduce intraocular pressure in the eye and therefore provide yet one more approach to the treatment of ocular hypertension and the degenerative ocular conditions related thereto. Blockage of potassium channels can diminish fluid secretion and, under some circumstances, increase smooth muscle contraction and would be expected to lower IOP and have neuroprotective effects in the eye [US5,573,758 and US5,925,342; Moore, et al., Invest. Ophthalmol. Vis. Sci., 38, 1997; WO89/10757, WO94/28900 and WO96/33719].

In addition, the combination of prostaglandin compounds of the F-series (PGF) with antimicrobial peptides has been used in the treatment of increased intraocular pressure, such as that caused by glaucoma and the reduction of ocular hypertension [US2006264353].

In spite of the different therapies that have been developed for lowering intraocular pressure, and especially glaucoma, many individuals do not respond well when treated with existing glaucoma therapies.

On the other hand, inflammatory bowel disease (IBD) is the generic term for a disease of an unknown cause that produces chronic inflammation or ulceration of the mucosa of the large and small intestine. This inflammatory bowel disease includes such diseases as ulcerative colitis and Crohn's disease.

The presently available medical treatments for IBD generally involve drug therapy directed towards the suppression of gastrointestinal inflammation. The most commonly used medicaments to treat IBD are anti-inflammatory drugs such as salicylates. The salycilate preparations may be effective in treating mild to moderate disease. Examples of salicylates include sulfasalazine, olsalazine and mesalamine. All of these medicaments are given orally in high doses for maximal therapeutic benefit. These medicaments are not without side effects including heartburn, nausea, vomiting, diarrhea and headache. People with more severe IBD can be treated with corticosteroids, such as prednisone and hydrocortisone, which are more potent and faster-acting than salicylates in the treatment of IBD, but are endowed with potential side effects. In IBD patients that do not response to salicylates or corticosteroids, medicaments that suppress the immune system are used. However, immunosuppressants cause increase risk of infection, renal failure, and may increase the need for hospitalization. Drugs like antidiarrheals, laxatives and pain relievers can be also given to help relieve symptoms. Since all the available medical treatments for IBD are rather unsatisfactory and often ineffective, there is currently a great need for novel drugs capable of treating IBD and preventing relapse.

Disulfide, sulfide, sulfoxide and sulfone derivatives of dianhydrohexite mononitrate have been used or evaluated in various studies related to different pathological conditions mediated by defects in the NO pathway, such as cardiovascular disorders [WO00/20420 and W02005/037842]. However, with all of these studies, there has been no recognition that these compounds are capable of being effective in the treatment of glaucoma and/or lowering intraocular pressure or being effective in the treatment of intestinal disorders, such as intestinal inflammatory.

### BRIEF DESCRIPTION OF THE INVENTION

The inventors have found surprisingly that compounds of formula (I), and specially 2-acetylthioisosorbide-5-mononitrate, have potential therapeutic effect against ophthalmological diseases, more precisely glaucoma, and intestinal disorders, more specifically intestinal inflammation.

The new application of these compounds is based on the results obtained by *in* vivo experiments in animals subjected to different stimulus such as intestinal inflammation and intra ocular pressure, wherein it has been observed that administration of compounds of formula (I) reduces significantly these adverse effects. Additionally, in the case of the treatment of ocular hypertension, the results pointed out that the compounds of formula (I) can be used even at concentration 10-fold lower than those used with conventional medicaments. Therefore, these compounds have a great efficacy in the reduction of ocular hypertension or intestinal disorders caused by different stimulus.

Accordingly, the present invention relates to the use of a compound of formula (I) or a tautomer, a pharmaceutically acceptable salt, a prodrug or a solvate thereof: 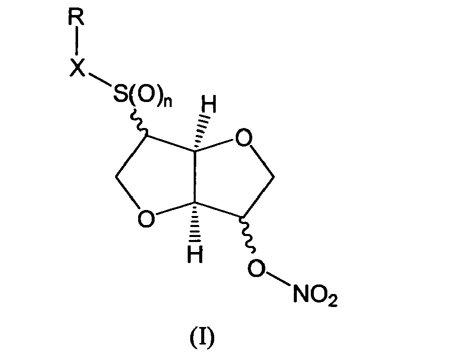 wherein:
n is an integer selected from 0, 1 and 2;
X is -S(O)ₘ-, -(C=O)- or a single bond, wherein m is an integer selected form 0, 1 and 2, with the proviso that when X is -(C=O)- then n is 0;
R is hydrogen or a residue R^{a}, wherein R^{a} is selected from the group consisting of:
   C₁₋₆ alkyl;
   C₂₋₆ alkenyl;
   C₃₋₈ cycloalkyl;
   C₃₋₈ cycloalkyl, wherein one CH₂ group is replaced by O, S, NH or NCH₃;
   C₄₋₈ cycloalkenyl;
   phenyl;
   pyridyl;
   thiophenyl;
   nitrosyl;
   S-cysteinyl;
   S-glutathionyl; and 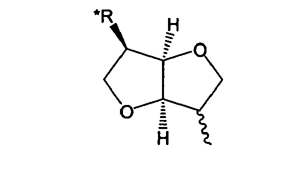 wherein R* is selected from the group consisting of hydrogen, C₁₋₆ alkyl, C₂₋₆ alkenyl, C₃₋₈ cycloalkyl, C₄₋₈ cycloalkenyl, acetyloxy, hydroxyl, ONO₂ and halogen;
   and wherein R^{a} is optionally substituted by one to three groups independently selected from C₁₋₆ alkyl, C₂₋₆ alkenyl, C₃₋₈ cycloalkyl, C₄₋₈ cycloalkenyl, acetyloxy, hydroxyl, ONO₂ and halogen,
as active ingredient(s) in the manufacture of a pharmaceutical composition for the prevention and/or treatment of an ophthalmological disease mediated by ocular hypertension.

In a particular embodiment, the ophthalmological disease is glaucoma, macular edema, age-related macular degeneration or diabetic retinopathy. In a preferred embodiment the ophthalmological disease is glaucoma.

Additionally, the present invention relates to the use of a compound of formula (I) as define above or a tautomer, a pharmaceutically acceptable salt, a prodrug or a solvate thereof as active ingredient(s) in the manufacture of a pharmaceutical composition for the prevention and/or treatment of an intestinal disorder.

In a particular embodiment, the intestinal disorder is intestinal inflammation.

In another aspect, the present invention refers to a method of preventing and/or treating an ophthalmological disease mediated by ocular hypertension, comprising administering to a patient in need thereof a therapeutically effective amount of a compound of formula (I) as defined above or a tautomer, a pharmaceutically acceptable salt, a prodrug or a solvate thereof.

Finally, another aspect of the present invention relates to a method of preventing and/or treating an intestinal disorder, comprising administering to a patient in need thereof a therapeutically effective amount of a compound of formula (I) as defined above or a tautomer, a pharmaceutically acceptable salt, a prodrug or a solvate thereof.

### DETAILED DESCRIPTION OF THE INVENTION

In the above definition of compounds of formula (I) used in the present invention, the following terms have the meaning indicated:
"C₁₋₆ alkyl" as used herein refers to a straight or branched hydrocarbon chain radical consisting of carbon and hydrogen atoms, containing no insaturation, having one to six carbon atoms, and which is attached to the rest of the molecule by a single bond, e. g., methyl, ethyl, n-propyl, i-propyl, n-butyl, t-butyl, n-pentyl, etc.
"C₂₋₆ alkenyl" as used herein refers to a straight or branched chain alkenyl moiety consisting of carbon and hydrogen atoms, having one to six carbon atoms and at least one double bond of either E or Z stereochemistry where applicable, e.g., vinyl, allyl, 1- and 2-butenyl, and 2-methyl-2-propenyl.
"C₃₋₈ cycloalkyl" as used herein refers to an alicyclic group consisting of carbon and hydrogen atoms, having three to eight carbon atoms, e.g., cyclopropyl, cyclobutyl, cyclopentyl and cyclohexyl.

Accordingly, the term "C₃₋₈ cycloalkyl wherein one CH₂ group is replaced by O, S, NH or NCH₃" as used herein refers to an alicyclic group having from three to eight carbon atoms wherein one CH₂ group is replaced by O, S, NH o NCH₃, e.g., tetrahydropyrane, tetrahydrofurane, pyrrolidine, piperidine and tetrahydrothiophene.

"C₄₋₈ cycloalkenyl" as used herein refers to an alicyclic group consisting of carbon and hydrogen atoms, having four to eight carbon atoms, e.g., cyclopentenyl, cyclohexenyl, cycloheptenyl and cyclooctenyl.

"Halogen" as used herein refers to fluorine, chlorine, bromine or iodine, whereof bromine is preferred.

It is preferred that R represents hydrogen C₁₋₆ alkyl, C₂₋₆ alkenyl, C₃₋₈ cycloalkyl, C₄₋₈ cycloalkenyl, (C₁₋₆ alkyl)C₃₋₈ cycloalkyl, (C₁₋₆ alkyl)C₄₋₈ cycloalkenyl, phenyl or (C₁₋₆ alkyl)phenyl, whereas C₁₋₆ alkyl is especially preferred.

It is further preferred that in formula (I) either one or both of m and n is 0.

Also it is preferred that X represents a single bond or -S-.

It is especially preferred that in the compounds of formula (I), RXS(O)ₙ- and ONO₂ are trans to each other with respect to the ring plane. The compound of formula (I) also include (R) and (S) diastereoisomers according to the formula (Ia) and (Ib):

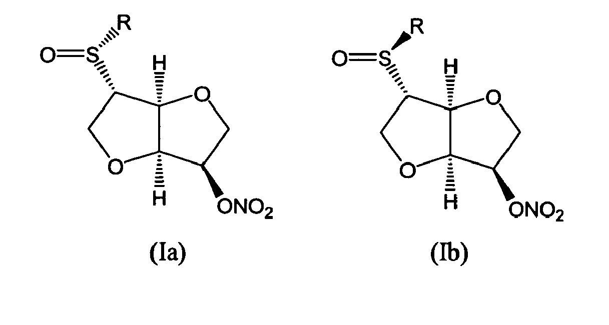

Especially preferred compounds of formula (I) are:
2-thioisosorbide 5-mononitrate;
5,5'-dinitrate-2,2'-dithiodiisosorbide;
2-methylthioisosorbide 5-mononitrate;
2-[(R)-methylsulfinyl]isosorbide 5-mononitrate;
2-[(S)-methylsulfinyl]isosorbide 5-mononitrate;
2-methylsulfinylisosorbide 5-mononitrate;
2-methylsulfonylisosorbide 5-mononitrate;
S-nitroso-2-thioisosorbide 5-mononitrate;
2-(tetrahydropyran-2-yl-thio) isosorbide 5-mononitrate;
2-(isosorbidyl-2'-dithio) isosorbide 5-mononitrate; and
2-(5'-acetyloxyisosorbidyl-2'-dithio) isosorbide 5-mononitrate.

Further it is especially preferred to use 2-acetylthio-isosorbide-5-mononitrate: 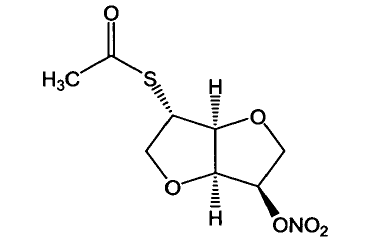 a tautomer, a pharmaceutically acceptable salt, a prodrug or a solvate thereof as active ingredient for the manufacture of a pharmaceutical composition for the prevention and/or treatment of an opthalmological disease mediated by ocular hypertension or an intestinal disorder.

In a particular embodiment the ophthalmological disease is glaucoma. In another particular embodiment the intestinal disorder is intestinal inflammation.

Unless otherwise stated, the compounds used in the invention are also meant to include compounds which differ only in the presence of one or more isotopically enriched atoms. For example, compounds having the present structures except for the replacement of a hydrogen by a deuterium or tritium, or the replacement of a carbon by a ¹³C- or ¹⁴C-enriched carbon or ¹⁵N-enriched nitrogen are within the scope of this invention.

The term "pharmaceutically acceptable salts, solvates, prodrugs" refers to any pharmaceutically acceptable salt, ester, solvate, or any other compound which, upon administration to the recipient is capable of providing (directly or indirectly) a compound as described herein. However, it will be appreciated that non-pharmaceutically acceptable salts also fall within the scope of the invention since those may be useful in the preparation of pharmaceutically acceptable salts. The preparation of salts, prodrugs and derivatives can be carried out by methods known in the art.

For instance, pharmaceutically acceptable salts of compounds used in the invention are synthesized from the parent compound which contains a basic or acidic moiety by conventional chemical methods. Generally, such salts are, for example, prepared by reacting the free acid or base forms of these compounds with a stoichiometric amount of the appropriate base or acid in water or in an organic solvent or in a mixture of the two. Generally, nonaqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p-toluenesulphonate. Examples of the alkali addition salts include inorganic salts such as, for example, sodium, potassium, calcium, ammonium, magnesium, aluminium and lithium salts, and organic alkali salts such as, for example, ethylenediamine, ethanolamine, N,N-dialkylenethanolamine, triethanolamine, glucamine and basic aminoacids salts.

Particularly favoured derivatives or prodrugs are those that increase the bioavailability of the compounds of this invention when such compounds are administered to a patient (e.g., by allowing an orally administered compound to be more readily absorbed into the blood) or which enhance delivery of the parent compound to a biological compartment (e.g., the brain or lymphatic system) relative to the parent species.

The term "prodrug" is used in its broadest sense and encompasses those derivatives that are converted in vivo to the compounds of the invention. Such derivatives would readily occur to those skilled in the art, and include, depending on the functional groups present in the molecule and without limitation, the following derivatives of the present compounds: esters, amino acid esters, phosphate esters, metal salts sulfonate esters, carbamates, and amides. Examples of well known methods of producing a prodrug of a given acting compound are known to those skilled in the art and can be found e.g. in Krogsgaard-Larsen et al. "Textbook of Drugdesign and Discovery" Taylor & Francis (april 2002).

The compounds used in the present invention may be in crystalline form either as free compounds or as solvates (e.g. hydrates).

The compounds of formula (I) or their salts or solvates used in the invention are preferably in pharmaceutically acceptable or substantially pure form. By pharmaceutically acceptable form is meant, "inter alia", having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I), or of its salts, solvates or prodrugs.

The compounds used in the present invention represented by the formula (I) can include enantiomers, depending on the presence of chiral centers, and/or depending on the presence of multiple bonds (for example Z, E). The pure isomers, enantiomers or diastereoisomers and their mixtures are within the scope of the present invention.

The compounds of formula (I) used in the invention can be obtained by available synthetic procedures. Some examples of these procedures are described in WO2005/037842 and references therein. The content of these documents is incorporated herein by reference in its entirety.

In a particular embodiment of the present invention, the compounds of formula (I) used in the treatment of ophthalmological diseases or intestinal disorders, are formulated in a suitable pharmaceutical composition, in a therapeutically effective quantity, together with one or more pharmaceutically acceptable carriers, adjuvants or excipients.

The pharmaceutical composition may be administered in the form of different preparations. Non limiting examples are preparations for oral administration, e.g. tablets, capsules, syrups or suspensions; ophthalmological administration, e.g. solutions, suspensions, ointments or creams; and parenteral administration, e.g. aqueous and non-aqueous sterile injection solutions or aqueous and non-aqueous sterile suspensions. Also, the pharmaceutical composition may include topical compositions, e.g. creams, ointments or pastes, or transdermic preparations such as patches or plasters. The pharmaceutical composition may also be prepared for vaginal or for rectal administration, e.g. rectal gel or suppository.

Generally an effective administered amount of a compound used in the invention will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.01 to 100 mg/kg/day.

The compounds used in the present invention may also be administered with other drugs to provide a combination therapy. The other drugs may form part of the same composition, or be provided as a separate composition for administration at the same time or at different time.

In a particular embodiment the invention refers to the use of a compound of formula (I) or a tautomer, a pharmaceutically acceptable salt, a prodrug or a solvate thereof, for the elaboration of a pharmaceutical composition for the treatment and/or prevention of an ophthamological disease mediated by ocular hypertension. In a preferred embodiment, the ophthamological disease is glaucoma, macular edema, age-related macular degeneration or diabetic retinopathy, more preferably is glaucoma.

In another particular embodiment the invention refers to the use of a compound of formula (I) or a tautomer, a pharmaceutically acceptable salt, a prodrug or a solvate thereof, for the elaboration of a pharmaceutical composition for the treatment and/or prevention of an intestinal disorder. In a preferred embodiment, the intestinal disorder is an intestinal inflammation. *In vivo* experiments in animals subjected to different stimulus such as high intraocular pressure and intestinal inflammation have shown that administration of compounds of formula (I), reduce significantly these adverse effects. Additionally, in the case of the treatment of glaucoma, the results pointed out that the compounds of formula (I) can be used even at concentration 10-fold lower than those used with conventional medicaments. Therefore, these compounds have a great efficacy in the reduction of glaucoma or intestinal disorders caused by different stimulus.

### EXAMPLES

### Example 1.- Results derived from the administration of 2-acetylthioisosorbide-5-mononitrate in mice which have been subjected to indomethacin-induced intestinal inflammation.

Intestinal inflammation were induced by administration of two subcutaneous injections of 7.5 mg/kg indomethacin, as previously described Porras M., Martin MT., Soler M. and Vergara P., "Intestinal motor disorders associated with cyclical bacterial overgrowth in a rat model of enteritis". Am. J. Physiol. Gastrointest. Liver Physiol. (2004), 287:G58-G64, and Porras M., Martin MT., Torres R. and Vergara P., "Cyclical upregulated iNOS and long-term downregulated nNOS are the bases for relapse and quiescent phases in a rat model of IBD". Am. J. Physiol. Gastrointest. Liver Physiol. (2006), 290:G423-G430.

Once the intestinal inflammation has been induced, one group of mice were treated with 30 mg of the compound 2-acetylthioisosorbide-5-mononitrate per kg of body weight, being dissolved this compound in water and administered orally. For comparative data, the compound isosorbide-5-mononitrate (IS-5-MN), structurally similar to 2-acetylthioisosorbide-5-mononitrate, was also administered to another group of mice.

Myeloperoxidase (MPO) activity was measured as an inflammation index in homogenized tissue samples after centrifugation using a specific ELISA kit (HyCult biotechnology, Uden, The Netherlands). In addition, since the barrier function of the intestinal mucus is deteriorated when the epithelium is inflamed and, as a consequence, the exposed bacteria cross the membrane and enter in the blood current, bacterial translocation was measured by detection of viable enteric bacteria in mesenteric lymph nodes as described by [M Mainous MR., Tso P., Berg R.D. and Deitch E.A., Arch Surg (1991) 126:33-37] as additional measure of inflammation degree.

Studies of the route, magnitude, and time course of bacterial translocation in a model of systemic inflammation were achieved as describe in Arch Surg 126:33-37, 1991. Bacterial translocation was expressed as the number of positive cultures with respect to the total number of samples in each group and motor activity was measured as the contracting activity expressed as the total number of spontaneous contractions recorded at duodenum per minute. Results are given in Table 1.

**Table 1**

| Group | Bacterial translocation | | MPO activity ng/ml | Contractions/min |
|---|---|---|---|---|
| | E. coli | Enterococcus sp | | |
| Control | 1/6 | 0/6 | 5±1 | 0.25±0.1 |
| Intestinal inflammation | 4/6 | 4/6 | 25±2 | 0.6±0.1 |
| IS-5-MN | 3/6 | 3/6 | 19±4 | 0.5±0.1 |
| 2-acetyl thioisosorbide-5-mononitrate | 2/6 | 1/6 | 10±2* | 0.2±0.1 |

These results point out that compound 2-acetylthioisosorbide-5-mononitrate leads to a significant reduction of MTO activity when compared to a structurally similar compound. In addition, the bacterial translocation is also reduced avoiding the entry of bacteria to the blood current, therefore confirming the utility of the compounds of the invention for treating intestinal inflammation.

### Example 2. Results derived from the administration of 2-acetylthioisosorbide-5-mononitrate and 2-[(R)-methylsulfinyl] isosorbide-5-mononitrate in rabbits which have been subjected to alfa-chymotrypsin-induced glaucoma.

The method used was that described by Gabriele Campana, Claudio Bucolo, Giovanna Murari and Santi Spampinato in Pharmacol. Exp Therap Vol. 303, Issue 3, 1086-1094, December 2002

Animals were injected with intra muscular injection of dexamethasone at the dose rate of 10 mg/Kg body weight, to avoid immediate inflammation. Animals were anesthetized with ketamine (50 mg/kg IV) in combination with Dizepam.

Xylocaine (4%) was used for local anesthesia topically. A cannula attached to reservoir was inserted into the anterior chamber with the help of a 30 gauge needle to provide a hydrostatic pressure of 25 mmHg during injection of alpha-chymotrypsin. Then a second appropriately shaped 30 gauge needle was introduced near the pupil. Freshly prepared 150 units of alpha chymotrypsin prepared in 0.1 ml of sterile saline was irrigated through the cannula into the posterior chamber. Care was taken to prevent the contact of alpha chymotrypsin with corneal stroma. Both cannulas were carefully removed without significant loss of aqueous humor. Immediately after surgery the eye treated with Sofracort (Corticosteroid) to reduce the chances of fungal or microbial infection.

All the animals were kept under observation for 5 days and after these five days the intraocular pressure (IOP) was measured daily with a Schiontz type indentation tonometer using 5.5 gm weights and by compilation of readings the maximum period required to achieve a stable increase in IOP was determined. It was found that 2-3 weeks were sufficient to achieve a stable increase in IOP. IOP was measured after 15 days for 3 consecutive days, every morning (at same time) to assure stable IOP. The rejection criteria in our study was the removal of those rabbits from the study which showed IOP< 30 mmHg.

### Treatment:

All the animals were closely observed for the development of glaucoma. Thirty animals showing the symptoms of glaucoma and with the intra ocular pressure more than 30 mmHg were selected for the present investigation.

Compounds 2-acetylthioisosorbide-5-mononitrate and 2-[(R)-methylsulfinyl] isosorbide-5-mononitrate (0.05%) were administered to two different groups of rabbits at a concentration of 0.05% by weight [0.05g in 100 mL of saline serum] by 3 drops instillation. For comparative data, the compound Timolol (Timoftol®) widely used for the treatment of glaucoma, was also administered to another group of rabbits at a concentration of 0.5% by weight.

The intraocular pressure measurements are included in the table 2 in mm Hg taken at 0, 1 and 2 h after 3 drops instillation.

| **Group** | **Treatment** | **0 hr** | **1 hr** | **2 hr** |
|---|---|---|---|---|
| Gr I | **trans-2-acetyl thioisosorbide-5-mononitrate (0.05%)** | 34.33 ±0.68 | 24.72±0.47 | 23.86±0.32 |
| Gr II | **2-[(R)-methylsulfinyl] isosorbide-5-mononitrate (0.05%)** | 33.87±0.38 | 23.51 ±0.24 | 22.77±0.19 |
| Gr III | **Timolol 0.5%** | 34.45 ± 0.58 | 24.77±0.26 | 24.54±0.26 |
| control | **Normal saline** | 33.76±0.54 | 33.21 ±0.34 | 32.37±0.31 |

These results have pointed out that compounds 2-acetylthio-isosorbide-5-mononitrate and 2-[(R)-methylsulfinyl] isosorbide-5-mononitrate comprised in formula (I) significantly reduce the intraocular pressure showing similar results to those obtained with a conventional compound even when using 10-fold lower concentrations.

## Claims

1. Use of a compound of formula (I) or a tautomer, a pharmaceutically acceptable salt, a prodrug or a solvate thereof: 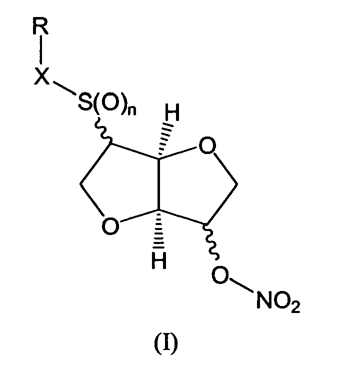 wherein:
n is an integer selected from 0, 1 and 2;
X is -S(O)ₘ-, -(C=O)- or a single bond, wherein m is an integer selected form 0, 1 and 2, with the proviso that when X is -(C=O)- then n is 0;
R is hydrogen or a residue R^{a}, wherein R^{a} is selected from the group consisting of:
C₁₋₆ alkyl;
C₂₋₆ alkenyl;
C₃₋₈ cycloalkyl;
C₃₋₈ cycloalkyl, wherein one CH₂ group is replaced by O, S, NH or NCH₃;
C₄₋₈ cycloalkenyl;
phenyl;
pyridyl;
thiophenyl;
nitrosyl;
S-cysteinyl;
S-glutathionyl; and 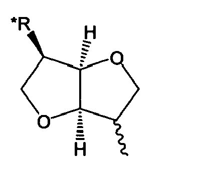 wherein R* is selected from the group consisting of hydrogen, C₁₋₆ alkyl, C₂₋₆ alkenyl, C₃₋₈ cycloalkyl, C₄₋₈ cycloalkenyl, acetyloxy, hydroxyl, ONO₂ and halogen;
and wherein R^{a} is optionally substituted by one to three groups independently selected from C₁₋₆ alkyl, C₂₋₆ alkenyl, C₃₋₈ cycloalkyl, C₄₋₈ cycloalkenyl, acetyloxy, hydroxyl, ONO₂ and halogen,
as active ingredient(s) in the manufacture of a pharmaceutical composition for the prevention and/or treatment of an ophthalmological disease mediated by ocular hypertension.

2. Use of a compound of formula (I) or a tautomer, a pharmaceutically acceptable salt, a prodrug or a solvate thereof: 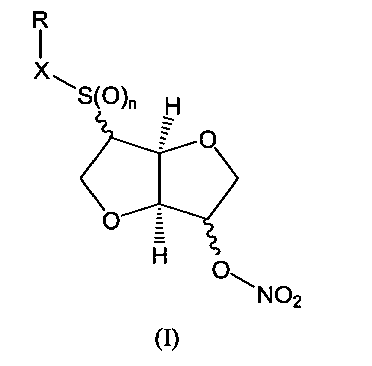 wherein:
n is an integer selected from 0, 1 and 2;
X is -S(O)m-, -(C=O)- or a single bond, wherein m is an integer selected form 0, 1 and 2, with the proviso that when X is -(C=O)- then n is 0;
R is hydrogen or a residue Ra, wherein Ra is selected from the group consisting of:
C₁₋₆ alkyl;
C₂₋₆ alkenyl;
C₃₋₈ cycloalkyl;
C₃₋₈ cycloalkyl, wherein one CH₂ group is replaced by O, S, NH or NCH₃;
C₄₋₈ cycloalkenyl;
phenyl;
pyridyl;
thiophenyl;
S-cysteinyl;
S-glutathionyl; and 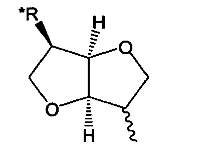 wherein R* is selected from the group consisting of hydrogen, C₁₋₆ alkyl, C₂₋₆ alkenyl, C₃₋₈ cycloalkyl, C₄₋₈ cycloalkenyl, acetyloxy, hydroxyl, ONO₂ and halogen;
and wherein R^{a} is optionally substituted by one to three groups independently selected from C₁₋₆ alkyl, C₂₋₆ alkenyl, C₃₋₈ cycloalkyl, C₄₋₈ cycloalkenyl, acetyloxy, hydroxyl, ONO₂ and halogen,
as active ingredient(s) in the manufacture of a pharmaceutical composition for the prevention and/or treatment of an intestinal disorder.

3. A method of preventing and/or treating an ophthalmological disease mediated by ocular hypertension, comprising administering to a patient in need thereof a therapeutically effective amount of a compound of formula (I) or a tautomer, a pharmaceutically acceptable salt, a prodrug or a solvate thereof: 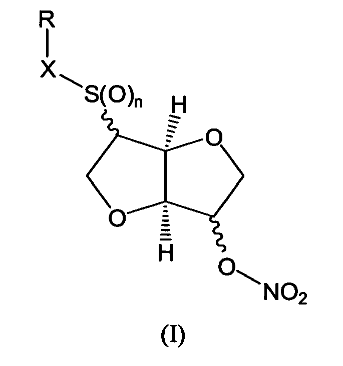 wherein:
n is an integer selected from 0, 1 and 2;
X is -S(O)m-, -(C=O)- or a single bond, wherein m is an integer selected form 0, 1 and 2, with the proviso that when X is -(C=O)- then n is 0;
R is hydrogen or a residue Ra, wherein Ra is selected from the group consisting of:
C₁₋₆ alkyl;
C₂₋₆ alkenyl;
C₃₋₈ cycloalkyl;
C₃₋₈ cycloalkyl, wherein one CH₂ group is replaced by O, S, NH or NCH₃;
C₄₋₈ cycloalkenyl;
phenyl;
pyridyl;
thiophenyl;
S-cysteinyl;
S-glutathionyl; and 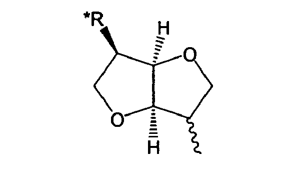 wherein R* is selected from the group consisting of hydrogen, C₁₋₆ alkyl, C₂₋₆ alkenyl, C₃₋₈ cycloalkyl, C₄₋₈ cycloalkenyl, acetyloxy, hydroxyl, ONO₂ and halogen;
and wherein R^{a} is optionally substituted by one to three groups independently selected from C₁₋₆ alkyl, C₂₋₆ alkenyl, C₃₋₈ cycloalkyl, C₄₋₈ cycloalkenyl, acetyloxy, hydroxyl, ONO₂ and halogen.

4. A method of preventing and/or treating an intestinal disorder, comprising administering to a patient in need thereof a therapeutically effective amount of a compound of formula (I) or a tautomer, a pharmaceutically acceptable salt, a prodrug or a solvate thereof: 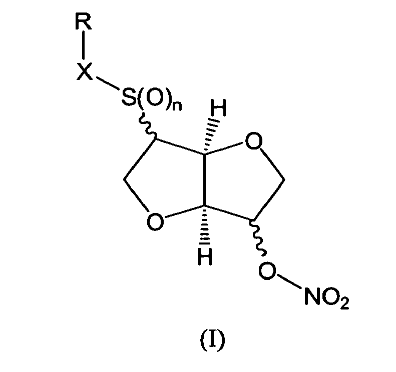 wherein:
n is an integer selected from 0, 1 and 2;
X is -S(O)m-, -(C=O)- or a single bond, wherein m is an integer selected form 0, 1 and 2, with the proviso that when X is -(C=O)- then n is 0;
R is hydrogen or a residue Ra, wherein Ra is selected from the group consisting of:
C₁₋₆ alkyl;
C₂₋₆ alkenyl;
C₃₋₈ cycloalkyl;
C₃₋₈ cycloalkyl, wherein one CH₂ group is replaced by O, S, NH or NCH₃;
C₄₋₈ cycloalkenyl;
phenyl;
pyridyl;
thiophenyl;
S-cysteinyl;
S-glutathionyl; and 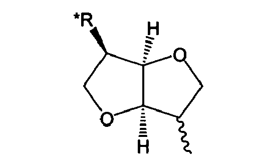 wherein R* is selected from the group consisting of hydrogen, C₁₋₆ alkyl, C₂₋₆ alkenyl, C₃₋₈ cycloalkyl, C₄₋₈ cycloalkenyl, acetyloxy, hydroxyl, ONO₂ and halogen;
and wherein R^{a} is optionally substituted by one to three groups independently selected from C₁₋₆ alkyl, C₂₋₆ alkenyl, C₃₋₈ cycloalkyl, C₄₋₈ cycloalkenyl, acetyloxy, hydroxyl, ONO₂ and halogen,

5. The use according to claims 1 or 3 wherein the ophthalmological disease is glaucoma, macular edema, age-related macular degeneration or diabetic retinopathy, more preferably is glaucoma.

6. The use according to claims 2 or 4 wherein the intestinal disorder is intestinal inflammation.

7. The use according to any one of claims 1 to 6 wherein either one or both of m and n is 0.

8. The use according to any one of claims 1 to 7 wherein X represents a single bond or -S-.

9. The use according to any one of claims 1 to 8 wherein R is hydrogen C₁₋₆ alkyl, C₂₋₆ alkenyl, C₃₋₈ cycloalkyl, C₄₋₈ cycloalkenyl, (C₁₋₆ alkyl)C₃₋₈ cycloalkyl, (C₁₋₆ alkyl)C₄₋₈ cycloalkenyl, phenyl or (C₁₋₆ alkyl)phenyl.

10. The use according to any one of claims 1 to 9 wherein R is C₁₋₆ alkyl.

11. The use according to any one of claims 1 to 10 wherein the compound according to formula (I) is a compound of formula (Ia) or (Ib):

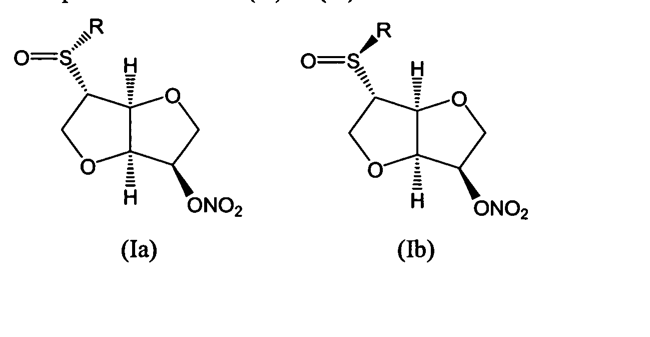

12. The use according to any one of claims 1 to 11 wherein the compound of formula (I) is selected from:
2-thioisosorbide 5-mononitrate;
5,5'-dinitrate-2,2'-dithiodiisosorbide;
2-methylthioisosorbide 5-mononitrate;
2-[(R)-methylsulfinyl]isosorbide 5-mononitrate;
2-[(S)-methylsulfinyl]isosorbide 5-mononitrate;
2-methylsulfinylisosorbide 5-mononitrate;
2-methylsulfonylisosorbide 5-mononitrate;
S-nitroso-2-thioisosorbide 5-mononitrate;
2-(tetrahydropyran-2-yl-thio) isosorbide 5-mononitrate;
2-(isosorbidyl-2'-dithio) isosorbide 5-mononitrate; and
2-(5'-acetyloxyisosorbidyl-2'-dithio) isosorbide 5-mononitrate.

13. The use according to any of claims 1 to 4 wherein the compound is 2-acetylthioisosorbide 5-mononitrate which is represented by the following formula:
